# EUROPEAN PATENT APPLICATION

(11) **EP 4 710 989 A1**
(43) Date of publication of application: **18.03.2026**
(21) Application number: 24803604.8
(22) Date of filing: 05.04.2024
(51) Int. Cl.: A61M 37/00

(54) **STAMP-TYPE MICRONEEDLE**

(30) Priority: 08.05.2023 KR 20230059041
(71) Applicant: Labnpeople Co.,Ltd., Yangju-si, Gyeonggi-do 11477 (KR)
(72) Inventor: CHO, Sung Youn, Uijeongbu-si Gyeonggi-do 11790 (KR); CHOI, Duk Su, Yangju-si Gyeonggi-do 11472 (KR); KWAK, Yong Kwon, Uijeongbu-si Gyeonggi-do 11698 (KR)
(74) Representative: Maiwald GmbH
(86) International application number: PCT/KR2024/004577
(87) International publication number: WO 2024/232548

(57) **Abstract**

The present invention comprises: a body; a pressing part protruding from the upper end of the body; a cartridge detachably coupled to the pressing part; a base plate part which is disposed between the cartridge and the pressing part, and which has a plurality of needle parts formed thereon; and a cap detachably coupled to the body while the cartridge and the pressing part are accommodated therein, wherein the needle parts formed on the base plate part are bent by the pressure transmitted from the pressing part so as to protrude from the cartridge.

## Description

### Technical Field

The disclosure relates to a stamp-type microneedle and, more particularly, to a stamp-type microneedle configured to be inserted into the skin of a user to deliver high-dose active ingredients or medication simply and effectively and enhance skin absorption rates.

### Background Art

In general, active ingredients or medication may be delivered into the body through oral administration, injection, or transdermal absorption.

Among these methods, oral administration, i.e., taking medication through the mouth, may cause side effects involving the central nervous system, such as headache, drowsiness, dizziness, and nervousness, as well as serious gastrointestinal side effects, such as nausea, indigestion, diarrhea, peptic ulcers, gastrointestinal bleeding, and perforation. Therefore, the use of oral administration is restricted to short-term therapies only.

In addition, the method of administering medication via injection involves inserting a needle through the skin to inject medication directly into the body, thereby resulting in rapid absorption. However, medication administration via injection requires professional administration (making self-administration difficult). Because needles that are several millimeters in diameter are typically used, pain during injection consequently causes poor compliance of the patient.

To eliminate the above-described side effects and limitations, transdermal drug delivery systems are being actively developed.

Typically, medication that is absorbed through the skin is formulated as liquid, cream, gel, or the like. However, due to the formulation characteristics, medication such as liquid, cream, or gel presents difficulties in dose control, and is sticky or causes skin contamination. In addition, most medication has the problem that only very small quantity is absorbed and the majority evaporates.

Therefore, a method of attaching a patch containing active ingredients or medication to the skin is used to deliver the medication subcutaneously.

The patch method, in which medication is absorbed through the skin, may prevent side effects associated with oral medication, such as gastrointestinal issues or interference from food, and eliminate the pain and discomfort of injections. Due to the convenience, the use of patch method has been gradually increasing recently.

However, the patch method has the problem of low medication absorption rates through the skin. Specifically, the major drawback of patches is that the low absorption rates of medication through the skin necessitate reducing the function of the skin as a permeation barrier. Generally, the absorption rates of medication through patches are known to be approximately 20%. To date, methods for improving absorption rates include chemical approaches of using transdermal absorption enhancers, prodrugs, and the like, as well as physical methods such as iontophoresis and electrophoresis.

However, even when these announced methods are used, improving medication absorption rates to a satisfactory level remains challenging. Therefore, excessive medication use is unavoidable to expect therapeutic effects when absorption rates are considered.

In particular, the types of skin absorption enhancers are determined depending on the formulation design or the physicochemical properties of the constituent components of an agent to be used. To be effective, a concentration equal to or greater than a selected level is desired. However, when skin absorption enhancers are added, it is not easy to prevent crystal formation over time. Disadvantageously, adhesive properties such as adhesion and cohesion may change in a manner that renders skin absorption enhancers to be unsuitable for use.

Furthermore, technologies using electricity have the drawback of requiring patches to incorporate batteries, electrodes, circuits, and other components, which may complicate product designs and increase sizes, thereby not only resulting in high costs but also generating large amounts of waste after use.

Accordingly, to enhance the skin absorption rates of active ingredients or medication, melt-processed polymer microneedles loaded with active ingredients or medication have been developed. However, the microneedles have limitations. The microneedles have low medication loading capacity, have poor skin penetration efficiency due to strength limitations of polymer, and break when attached at an angle rather than vertically to the skin. Thus, microneedles still have the disadvantage of being difficult to commercialize.

To address the above-described problems, a method of forming and arranging a plurality of microneedles on a thin bioabsorbable metal plate containing components beneficial to the human body and then attaching the resulting structure to the skin was proposed.

However, the problem is that it is difficult to form and arrange the microneedles on the limited planar area of the metal plate under conditions maximizing the delivery effect of active ingredients or medication

There is also the problem that the metal plate attached to the skin may easily detach from the skin surface due to the user movement. Therefore, an adhesive patch was devised to prevent the metal plate from detaching from the skin. However, this approach has the disadvantage of causing psychological discomfort to the user, as the adhesive patch and the metal plate must be kept tightly adhered to the skin for a selected period of time.

Furthermore, the microneedles are formed from extremely thin metal plates, which makes the microneedles prone to breaking or bending when inserted into the skin.

Therefore, the applicant developed the present invention to solve the above-described problems. A related prior art document is US Patent Application Publication No. 2007-0293815, "Microprojection Array Application with Sculptured Microprojections for High Drug Loading."

### Disclosure

### Technical Problem

The disclosure has been made to solve the above-described problems, and an objective of the disclosure is to provide a stamp-type microneedle configured to allow a user to easily bend needles on a flat substrate before use.

Another objective of the disclosure is to provide a stamp-type microneedle configured to enable sterile packaging of a substrate portion on which the needles are arranged.

Another objective of the disclosure is to provide a stamp-type microneedle configured to allow an applicator operated by a user to be used semi-permanently.

Another objective of the disclosure is to provide a stamp-type microneedle configured to effectively deliver active ingredients or medication into the subcutaneous tissue of the user without requiring the substrate provided with the needles to be attached to the skin of the user for a selected period of time, and especially, to deliver a high dose of active ingredients or medication.

### Technical Solution

A stamp-type microneedle according to the disclosure includes: a pressing portion provided on an upper end of a body; a cartridge detachably coupled to the pressing portion; and a substrate disposed between the cartridge and the pressing portion and including a plurality of needles, wherein the needles provided on the substrate are bent under pressure transmitted from the pressing portion and protrude from an upper portion of the cartridge.

The pressing portion may include protrusions provided on an upper surface thereof to press a portion where the needles are provided in an entire region of the substrate, and the pressing portion includes coupling protrusions provided on side surfaces thereof to be coupled to the cartridge.

The cartridge may include: a holder configured to accommodate the substrate, disposed on an upper portion of the pressing portion to be capable of linearly reciprocating, and having openings which allow the needles or the protrusions to be inserted into and extend through; elastic pieces connected to longitudinal ends of the holder, capable of elastically contacting an upper surface of the body, and having a selected curvature; and coupling pieces connected to width-direction ends of the holder and fastened to the coupling protrusions provided on the side surfaces of the pressing portion.

The coupling pieces may include coupling holes which allow the coupling protrusions provided on the side surfaces of the pressing portion to be inserted thereinto.

The cartridge may further include handles connected to ends of the coupling pieces and protruding outward in a width direction of the holder.

The stamp-type microneedle may include a cap detachably coupled to the body while accommodating the cartridge and the pressing portion, wherein the cap includes a jig recess on a ceiling surface thereof, the jig recess allowing the cartridge to be inserted into and seated in.

The jig recess may include: a first recess allowing the holder of the cartridge to be inserted into and seated in; and second recesses provided at a location where the first recess is provided, with a larger depth than the first recess, wherein the protrusions of the pressing portion are configured to be inserted into the second grooves through the openings.

The substrate may be inserted into and seated in an inner space of the holder which is seated in the jig recess.

The portion where the needle portions are provided in the entire region of the substrate which is seated in the holder may be bent under pressure from the pressing portion which is inserted into an inner space of the cap and coupled to the cartridge.

In addition, when the holder of the cartridge contacts a skin of a user, a gap between an upper end of the holder and an upper end of the pressing portion may decrease, and at a same time, the needle portions of the substrate may be inserted into the skin of the user through the openings of the holder.

### Advantageous Effects

Because the stamp-type microneedle according to the disclosure allows consumers to easily bend the needles into a flat plate shape prior to use, the substrate may be packaged in a flat plate shape, thereby reducing a packaging volume and simplifying packaging operations. Furthermore, a plurality of substrates may be provided to consumers in a sterile state.

In addition, in the stamp-type microneedle according to the disclosure, the cap may prevent the pressing portion, the cartridge, and the substrate interposed between the pressing portion and the cartridge from being contaminated by an external source. At the same time, the cap may function as a jig which enables the needles 20 of the substrate to be banded. Thus, the consumer may replace only the substrate and semi-permanently use the remaining component.

In addition, the stamp-type microneedle according to the disclosure controls the linear reciprocating movement of the pressing portion using the elastic pieces integrally connected to the cartridge. This may eliminate the need for separate elastic means such as springs, thereby reducing the number of components and consequently lowering manufacturing costs.

In addition, the stamp-type microneedle according to the disclosure contains a bioabsorbable metallic component that provides beneficial effects to the skin upon insertion. Therefore, the stamp-type microneedle may provide skin stimulation and medication delivery effects, as well as a swelling effect in the subcutaneous tissue, and function as a medication delivery enhancer.

Furthermore, the stamp-type microneedle according to the disclosure provides a structure that allows the microneedles inserted into the skin to be easily replaced for each user, thereby preventing infection.

### Description of Drawings

FIG. 1 is a perspective view illustrating a stamp-type microneedle according to an embodiment of the disclosure;
FIG. 2 is an exploded perspective view illustrating the stamp-type microneedle shown in FIG. 1;
FIG. 3 is a perspective view of the substrate according to an embodiment of the disclosure;
FIG. 4 is a perspective view illustrating the bent state of the needles in the substrate shown in FIG. 3;
FIG. 5 is a perspective view illustrating the body and the pressing portion according to an embodiment of the disclosure;
FIG. 6 is a perspective view illustrating the cartridge according to an embodiment of the disclosure;
FIG. 7 is a perspective view illustrating the pressing portion of the body shown in FIG. 5, with the cartridge mounted thereon;
FIG. 8 is a bottom perspective view illustrating the interior of the cap according to an embodiment of the disclosure;
FIG. 9 is a bottom view of the cap according to an embodiment of the disclosure;
FIG. 10 is a perspective view illustrating the cap shown in FIGS. 8 and 9, with the cartridge seated in the jig recess of the cap;
FIG. 11 is a bottom view of the cap shown in FIG. 10;
FIG. 12 is a bottom view illustrating the cartridge shown in FIGS. 10 and 11, with the flat plate-shaped substrate shown in FIG. 3 seated in the cartridge; and
FIG. 13 is a cross-sectional view illustrating insertion of the body into the cap to bend the needles of the substrate shown in FIG. 12.

### Mode for Invention

Advantages and features of the disclosure, as well as methods of realizing the same, will be more clearly understood from the following detailed description of embodiments when taken in conjunction with the accompanying drawings.

However, the disclosure is not limited to specific embodiments described hereinafter but may be embodied in a variety of different forms. Rather, these embodiments are provided so that the description of the disclosure will be complete and will fully convey the scope of the disclosure to a person having ordinary skill in the technical field to which the disclosure pertains. The disclosure shall be defined by the scope of the Claims.

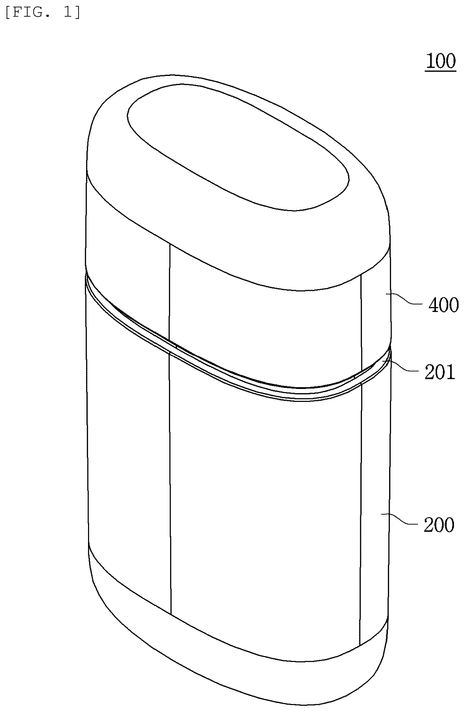

Hereinafter, a stamp-type microneedle according to an embodiment of the disclosure will be described in detail with reference to FIGS. 1 to 13. In describing the disclosure, specific descriptions of related known functions or configurations are omitted to avoid obscuring the essence of the disclosure.

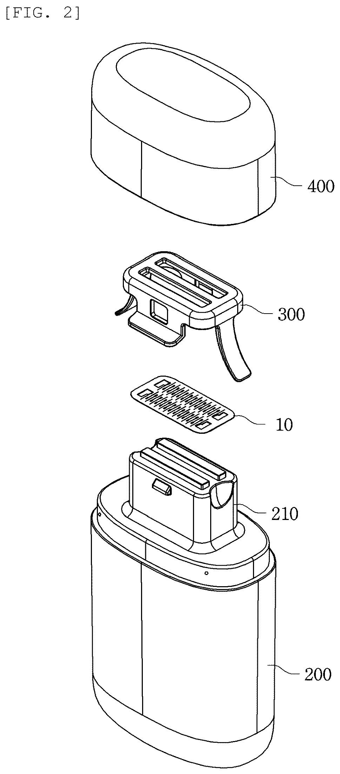

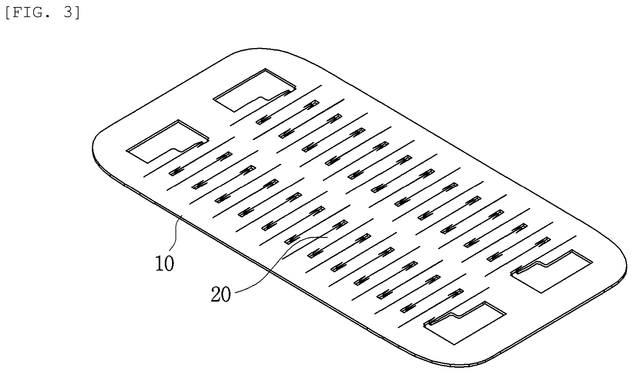

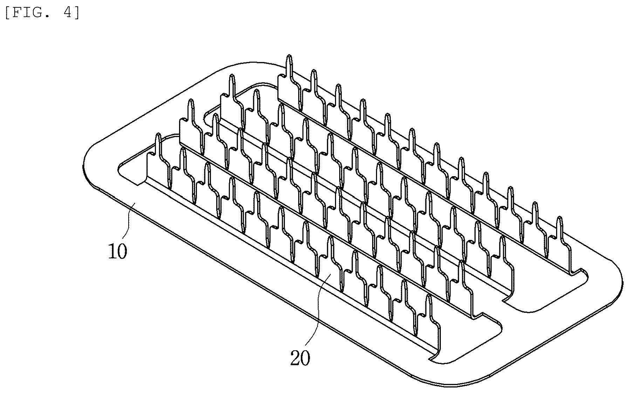

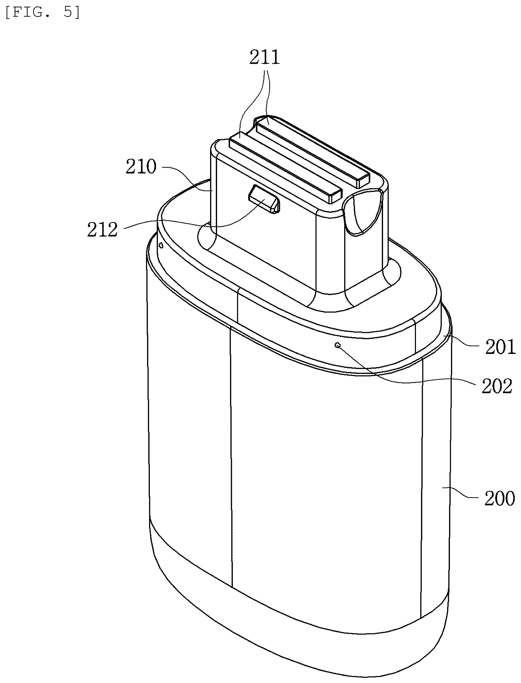

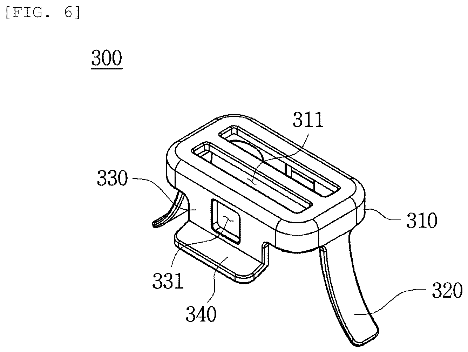

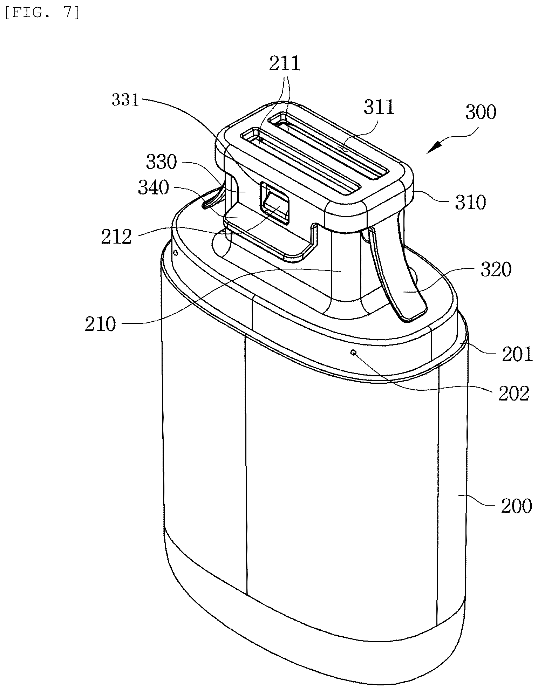

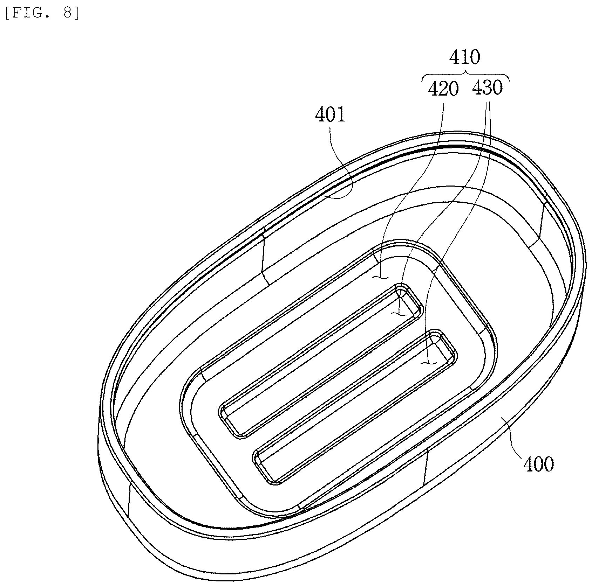

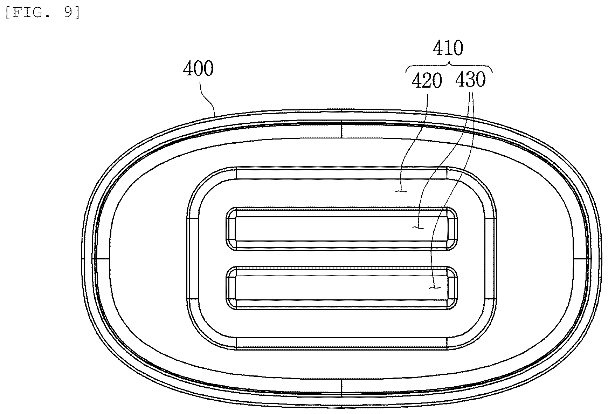

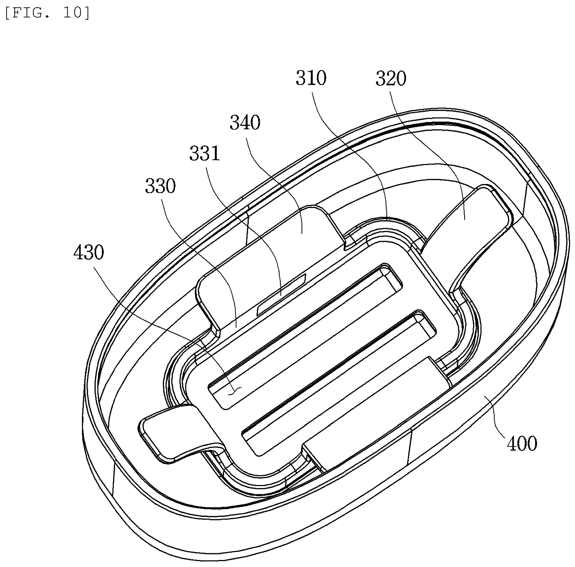

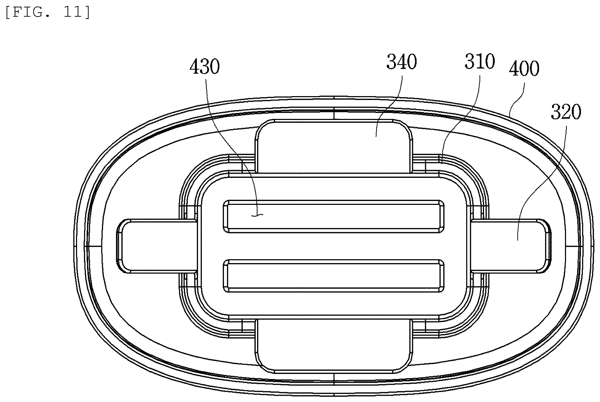

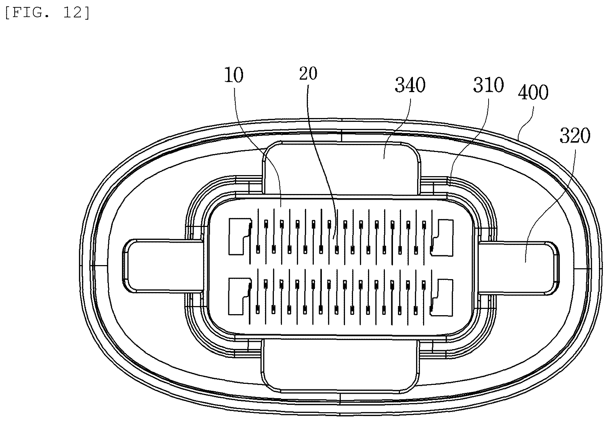

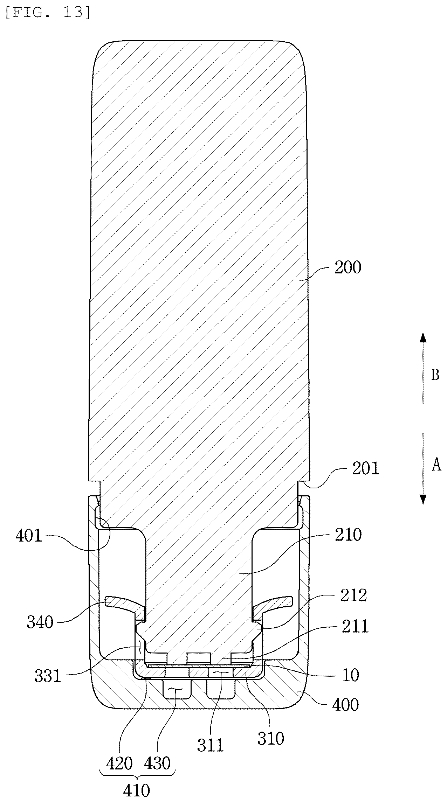

FIG. 1 is a perspective view illustrating a stamp-type microneedle according to an embodiment of the disclosure, FIG. 2 is an exploded perspective view illustrating the stamp-type microneedle shown in FIG. 1, FIG. 3 is a perspective view of the substrate according to an embodiment of the disclosure, FIG. 4 is a perspective view illustrating the bent state of the needles in the substrate shown in FIG. 3, FIG. 5 is a perspective view illustrating the body and the pressing portion according to an embodiment of the disclosure, FIG. 6 is a perspective view illustrating the cartridge according to an embodiment of the disclosure, FIG. 7 is a perspective view illustrating the pressing portion of the body shown in FIG. 5, with the cartridge mounted thereon, FIG. 8 is a bottom perspective view illustrating the interior of the cap according to an embodiment of the disclosure, FIG. 9 is a bottom view of the cap according to an embodiment of the disclosure, FIG. 10 is a perspective view illustrating the cap shown in FIGS. 8 and 9, with the cartridge seated in the jig recess of the cap, FIG. 11 is a bottom view of the cap shown in FIG. 10, FIG. 12 is a bottom view illustrating the cartridge shown in FIGS. 10 and 11, with the flat plate-shaped substrate shown in FIG. 3 seated in the cartridge, and FIG. 13 is a cross-sectional view illustrating insertion of the body into the cap to bend the needles of the substrate shown in FIG. 12.

As shown in FIGS. 1 and 2, the stamp-type microneedle 100 according to an embodiment of the disclosure includes: a body 200; a pressing portion 210 protruding from the upper end of the body 200; a cartridge 300 detachably coupled to the pressing portion 210; a substrate 10 disposed between the cartridge 300 and the pressing portion 210 and including a plurality of needles 20 provided thereon; and a cap 400 detachably coupled to the body 200 while accommodating the cartridge 300 and the pressing portion 210.

The body 200 may be considered a component held by the user. Therefore, the body 200 may have a size and shape that is easy to hold in the hand.

In addition, as shown in FIG. 5, a stepped portion 201 may be provided on the circumferential surface of the upper end portion of the body 200. The stepped portion 201 may be considered a region that contacts the lower end of the cap 400 described later and act as a stopper that limits the distance by which the body 200 moves in the cap 400.

The pressing portion 210 may be formed to protrude upward from the central upper portion of the body 200 and have a smaller planar area than the planar area of the body 200.

In addition, as shown in FIG. 5, protrusions 211 may be provided on the upper surface of the pressing portion 210 to press the portion where the needles 20 are provided in the entire region of the substrate 10. Furthermore, coupling protrusions 212 may be provided on side surfaces of the pressing portion 210 to be coupled to the cartridge 300.

The pressing portion 210 configured as described above serves to apply pressure to a portion of the area of the substrate 10 described later, thereby bending the needles 20 provided on the substrate 10 in a vertical direction. The pressing portion also supports the cartridge 300 described later so that the cartridge may linearly reciprocate, thereby allowing the needles 20 to be inserted into the skin of the user.

As shown in FIGS. 6 and 7, the cartridge 300 includes: a holder 310 having a shape capable of accommodating the substrate 10 (see FIG. 3), disposed on the upper portion of the pressing portion 200 to be capable of linearly reciprocating, and having openings 311 which allow the needles 20 (see FIG. 4) or the protrusions 211 to be inserted into and extend through; elastic pieces 320 connected to the longitudinal ends of the holder 310, capable of elastically contacting the upper surface of the body 200, and having a selected curvature; and coupling pieces 330 connected to width-direction ends of the holder 310 (i.e., ends of the holder 310 in the width direction) and fastened to the coupling protrusions 212 provided on the side surfaces of the pressing portion 200.

The holder 310 may have a shape that surrounds the entire circumference of the upper end portion of the pressing portion 210. When the coupling protrusion 212 of the pressing portion 200 is inserted into the coupling openings 311 provided in the coupling pieces 330, the holder may be disposed to be spaced apart from the upper surface of the pressing portion 210 by a selected distance.

In addition, the holder 310 may be inserted into a jig recess 410 (see FIG. 8) provided in the cap 400 described later and mounted on the cap 400.

In addition, the openings 311 provided in the holder 310 may have a shape conforming to the protrusions 211 provided on the upper surface of the pressing portion 210.

For reference, in an embodiment of the disclosure, the drawings show two protrusions 211 provided on the upper surface of the pressing portion 210, and two openings 311 are also provided in the holder 310 in a corresponding manner.

The holder 310 configured as described above may move toward the upper surface of the pressing portion 210 due to the force of the user pressing the pressing portion 210 of the body 200 against the skin. When the force of the user transmitted to the pressing portion 210 is released, the elastic pieces 320 may allow the holder to return to the original position.

The elastic pieces 320 may be integrally connected to the holder 310.

Specifically, one longitudinal end of each of the elastic pieces 320 may be integrally connected to one longitudinal end of the length of the holder 310. In addition, the other longitudinal ends of the elastic pieces 320 may extend toward the upper surface of the body 200.

Therefore, when the user presses the pressing portion 210 of the body 200 against the skin, the other longitudinal ends of the elastic pieces 320 may flex elastically while contacting the upper surface of the body 200. Conversely, when the force of the user transmitted to the pressing portion 210 is released, the elastic pieces may return to the original position due to elastic recovering force and allow the holder 310 to be upwardly spaced apart from the upper surface of the pressing portion 210.

The coupling pieces 330 may also be integrally connected to the holder 310.

Specifically, one longitudinal end of each of the coupling pieces 330 may be integrally connected to one width-direction end of the holder 310. In addition, the other longitudinal ends of the coupling pieces 330 may extend toward the upper surface of the body 200.

In addition, coupling holes 331 provided in the coupling pieces 330 may have a larger size than the coupling protrusions 212 provided on the side surfaces of the pressing portion 210 to allow the holder 310 to move.

Specifically, it is desirable that the coupling holes 331 be formed in the coupling pieces 330 with a space larger than the size of the coupling protrusion 212, so that the holder 310 may move in a direction against the pressing force when the pressing portion 210 presses against the skin. Therefore, as shown in FIG. 7, even when the coupling protrusions 212 are inserted into and engaged with the coupling holes 331, the coupling holes 331 retain a void space.

In addition, the cartridge 300 may further include handles 340 which are gripped by the user when separating the holder 310 from the pressing portion 210.

As shown in FIGS. 6 and 7, the handles 340 may be integrally connected to the coupling pieces 330. That is, one longitudinal end of each of the handles 340 may be integrally connected to the other longitudinal end of the corresponding coupling piece 330. In addition, the other longitudinal ends of the handles 340 may extend outward in the width direction of the holder 310.

Therefore, the user may spread the coupling pieces 330 outward in the width direction of the holder 310 using the handles 340. Then, the coupling protrusions 212 of the pressing portion 210 may be disengaged from the coupling holes 331 of the coupling pieces 330.

The cartridge 300 configured as described above serves as a guide to firmly support the substrate 10 described later, thereby enabling the needles 20 in a planar state to be easily bent vertically under pressure from the protrusions 211 of the pressing portion 210.

In addition, when the user presses the pressing portion 210 against the skin, the cartridge 300 exposes the bent needles 20, thereby enabling the needles to be inserted into the skin.

The substrate 10 may have a flat plate shape, as shown in FIG. 3.

In addition, the substrate 10 may be provided with a plurality of patterned needles 20.

The patterned needles 20 may be arranged in a flat plate shape, as shown in FIG. 3.

Therefore, because the substrate 10 has an overall flat plate shape, the substrate may be packaged sterile and provided to consumers.

Because the substrate 10 packaged in a flat plate shape is removed from the package to be used, the substrate may be disposed between the pressing portion 210 and the holder 310 of the cartridge 330, as shown in FIGS. 2 and 13.

The needles 20 of the substrate 10 disposed between the pressing portion 210 and the holder 310 may be bent into the shape shown in FIG. 4 by the pressing force transmitted from the pressing portion 210, thereby protruding from the upper portion of the cartridge 300. Specifically, the plurality of needles 20 provided on the substrate 10 may be bent by pressure applied from the protrusions 211 provided on the pressing portion 210 and then extend through the openings provided in the holder 310 to be exposed externally.

For reference, the substrate 10 may be formed from a metal containing at least one component selected from magnesium, calcium, zinc, and iron, which are used as bioabsorbable metals. Similarly, the needles 20 provided on the substrate 10 may also be formed from a metal containing at least one component selected from magnesium, calcium, zinc, and iron, which are used as bioabsorbable metals.

As a reference case, for the use of bioabsorbable metals in orthopedic implants, magnesium-based alloys were manufactured and commercialized both domestically and internationally. The use of bioabsorbable metals in orthopedic implants is focused on minimizing degradation rates in the body or enhancing corrosion resistance to ensure safe fracture fixation.

However, unlike bioabsorbable metals for orthopedic applications, the bioabsorbable metal forming the substrate 10 and the needles 20 according to an embodiment of the disclosure may use a mechanism that accelerates the degradation rate in the body to enable both medication release and mineral supply subcutaneously.

For example, bioabsorbable metals such as magnesium, calcium, and zinc may possess a mechanism whereby bioabsorbable metals react with water in the body to release hydrogen gas and decompose. Therefore, when the needles 20 provided on the substrate 10 are inserted into the skin, the needles release ions and decomposition products subcutaneously. One of these byproducts, hydrogen gas, may provide a swelling effect in the subcutaneous tissue, thereby inducing wrinkle improvement effects.

Furthermore, byproducts ZnO and MgCl, which are generated when magnesium and zinc, i.e., constituent elements of the bioabsorbable metal, are inserted into the body, may also function as medication delivery enhancers, which remain subcutaneously and improve the absorption of active ingredients or medication into the subcutaneous tissue. Therefore, the substrate 10 and the needles 20, which are formed from the bioabsorbable metal, also have the function of effectively delivering the active ingredients or medication contained therein, as well as active ingredients or medication delivered from outside, into the subcutaneous tissue.

For reference, the stainless steel used for microneedles has higher strength, elastic modulus, and melting point compared to magnesium. Therefore, when mechanically machining needles in a flat shape, more tool wear occurs and more energy is required, thereby increasing manufacturing costs. Even when a non-contact method such as laser processing is used, the high melting point demands more energy, generates heat, and causes burrs on the machined surface. Therefore, additional processes and costs for removing the burrs are required. In contrast, magnesium alloys have tensile strengths ranging from 100 to 400 MPa, which are sufficient to penetrate the skin, and elastic moduli of 40 to 60 GPa. Therefore, consumers need to apply significantly less bending force to magnesium alloys than to stainless steel. Magnesium having the melting point of 650 to 750°C may be processed with relatively low energy. The resulting surface is cleaner than stainless steel, thereby offering the advantage of being usable without post-processing. Furthermore, in a case in which needles inserted into the skin break or leave residues, stainless steel may cause inflammation or migration in the skin, thereby causing pain, necrosis, or the like. In contrast, magnesium alloys safely decompose and disappear.

The cap 400 may be detachably coupled to the upper end of the body 200, as shown in FIGS. 1 and 2.

The cap 400 also forms an inner space capable of accommodating the pressing portion 210 and the cartridge 300, as shown in FIGS. 8 and 9.

The cap 400 may be provided with the jig recess 410 on the ceiling surface. The jig recess is configured such that the cartridge 300 may be inserted into and seated in the jig recess.

The jig recess 410 may include: a first recess 420 allowing the holder 310 of the cartridge 300 to be inserted into and seated in; and second recesses 430 provided at the location where the first recess 420 is provided, with a larger depth than the first recess 420.

The first recess 410 may have a shape conforming to the holder 310 of the cartridge 300.

The second recesses 430 may have a shape conforming to the protrusions 211 of the pressing portion 210.

Therefore, as shown in FIG. 10 and FIG. 11, the user may insert the cartridge 300 into the inner space of the cap 400 so that the cartridge is seated in the jig recess 410.

As shown in FIGS. 10 and 11, the cartridge 300 is inserted into and fixed in the inner space of the cap 400 in order to provide a space allowing the flat substrate 10 to be seated therein and then apply pressure to and bend the needles 20 provided on the substrate 10.

Specifically, the substrate 10 may be inserted into and seated in the inner space of the holder 310 seated in the jig recess 410, as shown in FIG. 12.

In the substrate 10 seated in the holder 310, the portion where the needles 20 are provided may be inserted into the inner space of the cap 400, as shown in FIG. 13. The region of the substrate may be bent under pressure from the pressing portion 210 which engages with the cartridge 300.

Describing in detail, as shown in FIG. 13, the pressing portion 210 may be inserted into the inner space of the inverted cap 400 in the direction of arrow A to press the needles 20 of the substrate 10 seated in the holder 310. That is, after the protrusions 211 of the pressing portion 210 press the needles 20 seated in the holder 310, the protrusions may be inserted into the second recesses 430 of the jig recess 410.

Then, the needles 20 of the substrate 10, which had been in a flat plate shape, may be bent vertically by the protrusions 211. At the same time, the coupling protrusions 212 provided on the side surfaces of the pressing portion 210 may be inserted into the coupling holes 331 provided in the coupling pieces 330 of the cartridge 300.

In this position, when the force transmitted to the pressing portion 210 is released, the body 200 and the pressing portion 210 may be moved in the direction of arrow B shown in FIG. 13 by the elastic pieces 320 of the cartridge 300.

When this position is achieved, the needles 20 disposed between the pressing portion 210 and the holder 310 may be deformed into a shape capable of being inserted into the skin, as shown in FIG. 4.

The user may detach the cap 400 from the body 200 to insert the needles 20 into the skin. Thereafter, when the body 200 is pressed against the skin while the holder 310 of the cartridge 300 remains in contact with the skin, the gap between the upper end of the pressing portion 210 and the holder 310 decreases. At this time, the substrate 10 may be moved toward the position of the skin under pressure from the pressing portion 210. The bent needles 20 may be inserted into the skin through the openings 311 provided in the holder 310.

For reference, a border groove 401 may be provided on the inner circumferential surface of the cap 400, as shown in FIG. 13. The protrusions 202 (see FIG. 5) provided on the upper circumferential surface of the body 200 may be inserted into the border groove 401.

Because the stamp-type microneedle 100 having the above configuration allows consumers to easily bend the needles 20 into a flat plate shape prior to use, the substrate 10 may be packaged in a flat plate shape, thereby reducing a packaging volume and simplifying packaging operations. Furthermore, a plurality of substrates 10 may be provided to consumers in a sterile state.

In addition, in the stamp-type microneedle 100 according to the disclosure, the cap 400 prevents the pressing portion 210, the cartridge 300, and the substrate 10 interposed between the pressing portion and the cartridge from being contaminated by an external source. At the same time, the cap functions as a jig which enables the needles 20 of the substrate 10 to be banded. Thus, the consumer may replace only the substrate 10 and semi-permanently use the remaining component.

In addition, the stamp-type microneedle 100 according to the disclosure controls the linear reciprocating movement of the pressing portion 210 using the elastic pieces 320 integrally connected to the cartridge 300. This may eliminate the need for separate elastic means such as springs, thereby reducing the number of components and consequently lowering manufacturing costs.

Furthermore, although many people experience needle phobia, the concealed needles 20 of the stamp-type microneedle according to an embodiment of the disclosure do not cause fear, and the user feels pain after needles contact the skin. The user may properly control and use the stamp-type microneedle by applying an appropriate amount of force.

Moreover, to prevent the needles 20 from breaking upon penetration of the skin, the walls partitioning the openings 311 in the cartridge 300 may support the needles 20. Therefore, when used with active ingredients, the protrusions may apply pressure to the active ingredients between the needles after penetration, thereby significantly increasing skin absorption rates.

Although the specific embodiments of the disclosure have been described hereinabove, various modifications are possible without departing from the scope of the disclosure.

Therefore, the scope of the disclosure should not be limited to the described embodiments, but should be determined by the scope of the claims set forth below and the equivalents thereof.

### Industrial Applicability

The disclosure may be sold and used in various industrial fields, such as the medical field and the skin care field.

## Claims

1. A stamp-type microneedle comprising:
a pressing portion provided on an upper end of a body;
a cartridge detachably coupled to the pressing portion; and
a substrate disposed between the cartridge and the pressing portion and comprising a plurality of needles,
wherein the needles provided on the substrate are bent under pressure transmitted from the pressing portion and protrude from an upper portion of the cartridge.

2. The stamp-type microneedle of claim 1, wherein the pressing portion comprises protrusions provided on an upper surface thereof to press a portion where the needles are provided in an entire region of the substrate, and
the pressing portion comprises coupling protrusions provided on side surfaces thereof to be coupled to the cartridge.

3. The stamp-type microneedle of claim 2, wherein the cartridge comprises:
a holder configured to accommodate the substrate, disposed on an upper portion of the pressing portion to be capable of linearly reciprocating, and having openings which allow the needles or the protrusions to be inserted into and extend through;
elastic pieces connected to longitudinal ends of the holder, capable of elastically contacting an upper surface of the body, and having a selected curvature; and
coupling pieces connected to width-direction ends of the holder and fastened to the coupling protrusions provided on the side surfaces of the pressing portion.

4. The stamp-type microneedle of claim 3, wherein the coupling pieces comprise coupling holes which allow the coupling protrusions provided on the side surfaces of the pressing portion to be inserted thereinto.

5. The stamp-type microneedle of claim 3, wherein the cartridge further comprises handles connected to ends of the coupling pieces and protruding outward in a width direction of the holder.

6. The stamp-type microneedle of claim 3, further comprising a cap detachably coupled to the body while accommodating the cartridge and the pressing portion,
wherein the cap comprises a jig recess on a ceiling surface thereof, the jig recess allowing the cartridge to be inserted into and seated in.

7. The stamp-type microneedle of claim 6, wherein the jig recess comprises:
a first recess allowing the holder of the cartridge to be inserted into and seated in; and
second recesses provided at a location where the first recess is provided, with a larger depth than the first recess,
wherein the protrusions of the pressing portion are configured to be inserted into the second grooves through the openings.

8. The stamp-type microneedle of claim 7, wherein the substrate is inserted into and seated in an inner space of the holder which is seated in the jig recess.

9. The stamp-type microneedle of claim 8, wherein the portion where the needle portions are provided in the entire region of the substrate which is seated in the holder is bent under pressure from the pressing portion which is inserted into an inner space of the cap and coupled to the cartridge.

10. The stamp-type microneedle of claim 3, wherein when the holder of the cartridge contacts a skin of a user, a gap between an upper end of the holder and an upper end of the pressing portion decreases, and at a same time, the needle portions of the substrate are inserted into the skin of the user through the openings of the holder.
